# EUROPÄISCHE PATENTSCHRIFT

(11) **EP 1 919 483 B1**
(45) Veröffentlichungstag und Bekanntmachung des Hinweises auf die Patenterteilung: **30.12.2009**
(21) Anmeldenummer: 06791717.9
(22) Anmeldetag: 29.08.2006
(51) Int. Cl.: A61K 31/64, A61K 31/549, A61P 7/10

(54) **KOMBINATIONSPRÄPARAT AUS EINEM THIAZIDDIURETIKUM UND EINEM SCHLEIFENDIURETIKUM**
COMBINED PREPARATION OF A THIAZIDE DIURETIC AND A LOOP DIURETIC
PREPARATION COMBINEE D'UN DIURETIQUE DE THIAZIDE ET D'UN DIURETIQUE DE BOUCLE

(30) Priorität: 29.08.2005 DE 102005040841
(43) Veröffentlichungstag der Anmeldung: 14.05.2008
(73) Patentinhaber: Knauf, Heinrich, 79100 Freiburg (DE)
(72) Erfinder: KNAUF, Heinrich, 79100 Freiburg (DE); MUTSCHLER, Ernst, 55129 Mainz (DE)
(74) Vertreter: Winter, Brandl, Fürniss, Hübner Röss, Kaiser, Polte Partnerschaft Patent- und Rechtsanwaltskanzlei
(86) Internationale Anmeldenummer: PCT/EP2006/008455
(87) Internationale Veröffentlichungsnummer: WO 2007/025721

(56) Entgegenhaltungen:
- BRATER D C: "CLINICAL PHARMACOLOGY OF LOOP DIURETICS IN HEALTH AND DISEASE" EUROPEAN HEART JOURNAL, THE EUROPEAN SOCIETY OF CARDIOLOGY, XX, Bd. 13, Nr. SUPPL G, Dezember 1992 (1992-12), Seiten 10-14, XP009076528 ISSN: 0195-668X
- OSTER J R ET AL: "COMBINED THERAPY WITH THIAZIDE-TYPE AND LOOP DIURETIC AGENTS FOR RESISTANT SODIUM RETENTION" ANNALS OF INTERNAL MEDICINE, NEW YORK, NY, US, Bd. 99, Nr. 3, September 1983 (1983-09), Seiten 405-406, XP009076519 ISSN: 0003-4819 in der Anmeldung erwähnt
- BRATER D C: "PHARMACOLOGY OF DIURETICS" AMERICAN JOURNAL OF MEDICAL SCIENCES, XX, XX, Bd. 319, Nr. 1, Januar 2000 (2000-01), Seiten 38-50, XP009076524 ISSN: 0002-9629
- PAUL SARA: "BALANCING DIURETIC THERAPY IN HEART FAILURE: LOOP DIURETICS, THIAZIDES, AND ALDOSTERONE ANTAGONISTS" CONGESTIVE HEART FAILURE, CHF INC., GREENWICH, CT, US, Bd. 8, Nr. 6, November 2002 (2002-11), Seiten 307-312, XP009076526 ISSN: 1527-5299
- DORMANS T P ET AL: "COMBINATION DIURETIC THERAPY IN SEVERE CONGESTIVE HEART FAILURE" DRUGS, ADIS INTERNATIONAL LTD, AT, Bd. 55, Nr. 2, Februar 1998 (1998-02), Seiten 165-172, XP009076522 ISSN: 0012-6667
- WOLLAM, G,L, ET AL.: "Diuretic Potency of Combined Hydrochlorothiazide and Furosemide Therapy in Patients with Azotemia" THE AMERICAN JOURNAL OF MEDICINE, Bd. 72, Juni 1982 (1982-06), Seiten 929-938, XP002412903 usa in der Anmeldung erwähnt
- FLISER D ET AL: "COADMINISTRATION OF THIAZIDES INCREASES THE EFFICACY OF LOOP DIURETICS EVEN IN PATIENTS WITH ADVANCED RENAL FAILURE" KIDNEY INTERNATIONAL, NEW YORK, NY, US, Bd. 46, Nr. 2, August 1994 (1994-08), Seiten 482-488, XP009076520 ISSN: 0085-2538 in der Anmeldung erwähnt
- SICA D A ET AL: "DIURETIC COMBINATIONS IN REFRACTORY OEDEMA STATES: PHARMACOKINETIC-PHARMACODYNAMIC RELATIONSHIPS" CLINICAL PHARMACOKINETICS, LEA & FEBIGER, PHILADELPHIA, PA, US, Bd. 30, Nr. 3, März 1996 (1996-03), Seiten 229-249, XP009076525
- KNAUF H ET AL: "DIURETIC EFFECTIVENESS OF HYDROCHLOROTHIAZIDE AND FUROSEMIDE ALONE AND IN COMBINATION IN CHRONIC RENAL FAILURE" JOURNAL OF CARDIOVASCULAR PHARMACOLOGY, NEW YORK, NY, US, Bd. 26, Nr. 3, September 1995 (1995-09), Seiten 394-400, XP000991061 ISSN: 0160-2446 in der Anmeldung erwähnt
- SIGURD, B. ET AL.: "The supra-additive natriuretic effect addition of bendroflumethiazide and bumetanide in congestive heart failure" AMERICAN HEART JOURNAL, Bd. 89, Nr. 2, Februar 1975 (1975-02), Seiten 163-170, XP002412904 USA in der Anmeldung erwähnt

## Beschreibung

Die vorliegende Erfindung betrifft pharmazeutische Präparate, die eine fixe.Kombination aus einem niedrig dosierten Schleifendiuretikum und einem Thiaziddiuretikum enthalten.

Freie Schleifendiuretikum/Thiazid-Diuretikum-Kombinationen wurden - und werden - erfolgreich bei Patienten mit so genannter Diuretikaresistenz, d. h. bei Patienten, bei denen selbst hohe Dosen eines Schleifendiuretikums nicht oder nicht ausreichend wirksam sind, sowie bei Patienten mit schwerer Niereninsuffizienz eingesetzt. So wurde 1971 erstmals berichte, dass bei schwer herzkranken Patienten, die auf eine Hochdosis-Infusionstherapie mit Schleifendiuretika (z. B. Furosemid) nicht mehr ansprachen, nach zusätzlicher Gabe eines Thiaziddiuretikums (z. B. Chlorthalidon) die Diurese wieder in Gang kam. Bei schwer Nierenkranken kann durch die Diuretikakombination die sonst erforderliche sehr hohe Schleifendiuretikum-Dosis verringert und dadurch die Nebenwirkungsrate erniedrigt werden. In beiden Fällen wurde jedoch schon früh in der Literatur (1, 22) angemahnt, dass eine solche Behandlung wegen der Gefahr schwerwiegender Störungen des Wasser- und Elektrolythaushalts nur unter intensiver stationärer Kontrolle der Patienten in dafür speziell geschulten medizinischen Einrichtungen wie z. B. auf Intensivstationen oder in nephrologischen Zentren durchgeführt werden soll.

Der beschriebenen Kombinationsbehandlung liegt das Prinzip der sequentiellen Nephronblockade zugrunde. Darunter versteht man den Angriff der genannten Diuretika an verschiedenen, aufeinander folgenden Abschnitten des Tubulusapparats. Schleifendiuretika greifen, wie aus ihrem Namen hergeht, am dicken aufsteigenden Schenkel der Henle-Schleife an und hemmen hier den Na⁺/K⁺/2Cl⁻-Kotransporter, Thiazide blockieren dagegen weiter distal am frühdistalen Tubulus den Na⁺/Cl⁻-Kotransporter. Durch die Interaktion der Diuretika mit den Transportersystemen der Niere an zwei unterschiedlichen Stellen wird die Wirkung überproportional ("supraadditive Wirkung"; 2-27) verstärkt.

Während bei der eingangs geschilderten Akutbehandlung bedrohlicher Zustände die Therapie mit einem hochdosierten Schleifendiuretikum begonnen und erst bei ungenügender Wirkung mit zusätzlicher Gabe eines Thiazids weitergeführt wird, steht bei der Dauerbehandlung kardiovaskulärer Erkrankungen zunächst die Gabe eines Thiazids im Vordergrund, da speziell bei Hypertonie Thiazide sich im Vergleich mit Schleifendiuretika als wirksamer erwiesen haben (28, 29, 30). Zwar ist durch zahlreiche Studien belegt, dass durch eine solche Thiazid-Behandlung die Mortalität von Hochdruckkranken signifikant vermindert wird, doch hat auch sie Nachteile wie Kalium- und Magnesiumverluste sowie bei höheren Dosen Verschlechterung des Fett-, Zucker- und Harnsäurestoffwechsels.

Zunächst begegnete man den unerwünschten Elektrolytverlusten mit oraler Elektrolytsubstitution, die jedoch gerade von älteren Patienten schlecht vertragen wurde, und später mit der Einführung fixer Kombinationen aus einem Thiazid und einem Antikaliuretikum, z. B. Amilorid oder Triamteren. Doch auch diese Fixkombination erwies sich bei erhöhtem (sowie im Alter bei pseudonormalem) Plasmacreatinin wegen der Gefahr einer Hyperkaliämie als problematisch. Darüber hinaus ist bei Gabe eines Kaliumsparers zusammen mit einem Thiazid die häufig erforderliche zusätzliche Behandlung mit einem ACE-Hemmer bzw. einem AT₁-Antagonisten nicht mehr möglich.

Es wurde nun überraschend gefunden, dass durch die fixe Kombination eines Thiaziddiuretikums mit einem niedrig dosierten Schleifendiuretikum nicht nur die natriuretische/diuretische Wirkung insgesamt gesteigert, somit auch mit dieser niedrig dosierten Kombination ein supraadditiver Effekt erreicht wird, sondern auch die unerwünschten Nebenwirkungen bezüglich Kalium- und Magnesiumverlusten signifikant vermindert werden. Dabei bleibt trotz Anwesenheit des Schleifendiuretikums die erwünschte hypocalciurische Wirkung des Thiazids erhalten.

Gegenstand der vorliegenden Erfindung ist daher ein Kombinationspräparat, das als Wirkstoffe, zusammen oder getrennt voneinander, umfasst:
(a) ein Thiaziddiuretikum in einer Menge, die der Wirkung einer Dosis von 5 bis 50 mg Hydrochlorothiazid entspricht; und
(b) ein Schleifendiuretikum in einer Menge, die der Wirkung einer Dosis von 2,5 bis 15 mg Torasemid entspricht.

Das Kombinationspräparat der vorliegenden Erfindung stellt also eine fixe Wirkstoffkombination dar, bei der Auswahl und Dosis der Wirkstoffe festgelegt sind.

Das Kombinationspräparat kann die Wirkstoffe, die gewöhnlich in fester Form vorliegen, zusammen, also in Form einer einzigen Dosierungseinheit, oder getrennt voneinander, d.h. in Form individueller Dosierungseinheiten zur gemeinsamen Verabreichung, enthalten.

Unter Thiaziddiuretika, die in dem erfindungsgemäßen Kombinationspräparat verwendet werden können, werden Diuretika verstanden, die den Na⁺/Cl⁻-Carrier im distalen Tubulus hemmen und die Ausscheidung von Natrium-, Chlorid-, Kalium- und Magnesiumionen steigern. Hierzu gehören Thiazide und Thiazidanaloge. Geeignete Thiaziddiuretika sind beispielsweise Hydrochlorothiazid, Chlorothiazid, Benzthiazid, Cyclothiazid, Trichlormethiazid, Cyclopenthiazid, Methyclothiazid, Benzylhydrochlorothiazid, Ethiazid, Hydroflumethiazid, Polythiazid, Bendroflumethiazid, Chlorthalidon, Indapamid, Mefrusid, Metolazon, Bemetizid, Clopamid, Fenquizon, Quinethazon, Butizid, Xipamid und Indapamid, sowie pharmazeutisch verträgliche Derivate, beispielsweise Salze, davon. Bevorzugt wird Hydrochlorothiazid als Thiaziddiuretikum eingesetzt.

Unter Schleifendiuretika, die in dem erfindungsgemäßen Kombinationspräparat verwendet werden können, werden Diuretika verstanden, die, wie oben erwähnt, den Na⁺/K⁺/2Cl⁻-Carrier im aufsteigenden Schenkel der Henle-Schleife und dadurch die Resorption von Natrium-, Kalium- und Chloridionen hemmen. Geeignete Schleifendiuretika sind Torasemid, Furosemid, Azosemid, Bumetanid, Piretanid, Tripamid, Etozolin sowie dessen Metabolit Ozolinon, und Cicletanin, sowie pharmazeutisch verträgliche Derivate, beispielsweise Salze und Ester, davon. Bevorzugt wird Torasemid als Schleifendiuretikum eingesetzt.

Das erfindungsgemäße Kombinationspräparat enthält das Thiaziddiuretikum in der jeweiligen Dosierungseinheit in einer Menge, die der Wirkung einer Dosis von 5 bis 50 mg Hydrochlorothiazid entspricht. Besonders bevorzugt liegt das Thiaziddiuretikum in einer Menge vor, die einer Dosis von 5 bis 40 mg Hydrochlorothiazid entspricht, und ganz besonders bevorzugt ist eine Menge, die einer Dosis von 10 bis 30 mg entspricht. Übliche Mengen des Thiaziddiuretikums entsprechen beispielsweise einer Dosis von 12,5 und 25 mg Hydrochlorothiazid

Unter "Menge eines Thiaziddiuretikums, die der Wirkung einer Dosis von 5 bis 50 mg Hydrochlorothiazid entspricht", wird hier diejenige Menge Thiaziddiuretikum verstanden, die während der Wirkdauer dieses Thiaziddiuretikums die gleiche Menge NaCl zur Ausscheidung bringt wie 5 bis 50 mg Hydrochlorothiazid. Hierbei bedeutet "Wirkdauer" des Thiaziddiuretikums die Zeit, in der die wirkstoffbedingte NaCl-Ausscheidung über der Kontrollausscheidung ohne das Thiaziddiuretikum ("Baseline") liegt. Beispielsweise ist die Menge eines Thiaziddiuretikums, die der Wirkung einer Dosis von 25 mg Hydrochlorothiazid entspricht, eine Menge, die während der Wirkdauer dieses Thiaziddiuretikums die gleiche Menge NaCl zur Ausscheidung bringt wie 25 mg Hydrochlorothiazid. Das Wirkprofil von Diuretika wird beispielsweise bei Knauf und Mutschler (31) ausführlich beschrieben.

Dementsprechend werden Thiaziddiuretika wie Hydroflumethiazid, Benzthiazid, Chlorthalidon und Quinethazon gewöhnlich wie Hydrochlorothiazid in einer Menge von etwa 5 bis 50 mg, besonders bevorzugt von etwa 5 bis 40 mg und ganz besonders bevorzugt von etwa 10 bis 30 mg, beispielsweise 12,5 bis 25 mg, pro Dosierungseinheit eingesetzt. Bendroflumethiazid, Metolazon und Methyclothiazid liegen gewöhnlich in einer Menge von etwa 0,5 bis 5 mg, besonders bevorzugt von etwa 0,5 bis 4 mg und ganz besonders bevorzugt von etwa 1 bis 3,0 mg vor. Indapamid liegt gewöhnlich in einer Menge von etwa 0,25 bis 1,25 mg, besonders bevorzugt von etwa 0,25 bis 2 mg und ganz besonders bevorzugt von etwa 0,5 bis 1,5 mg vor. Polythiazid und Trichlormethiazid liegen gewöhnlich in einer Menge von 0,2 bis 2 mg, besonders bevorzugt von etwa 0,2 bis 1,6 mg und ganz besonders bevorzugt von etwa 0,4 bis 1,2 mg vor.

Das Schleifendiuretikum ist in dem erfindungsgemäßen Kombinationspräparat in der jeweiligen Dosierungseinheit in einer Menge enthalten, die der Wirkung einer Dosis von 2,5 bis 15 mg Torasemid entspricht. Bevorzugt liegt das Schleifendiuretikum in einer Menge vor, die einer Menge von 2,5 bis 14 mg entspricht, besonders bevorzugt in einer Menge von 2,5 bis 12,5 mg. Übliche Mengen des Schleifendiuretikums entsprechen beispielsweise einer Dosis von 2,5, 5 oder 10 mg Torasemid.

Unter "Menge eines Schleifendiuretikums, die der Wirkung einer Dosis von 2,5 bis 15 mg Torasemid entspricht", wird hier diejenige Menge Schleifendiuretikum verstanden, die während der Wirkdauer dieses Schleifendiuretikums die gleiche Menge NaCl zur Ausscheidung bringt wie 2,5 bis 15 mg Torasemid. Hierbei bedeutet "wirkdauer" des Schleifendiuretikums wie oben die Zeit, in der die wirkstoffbedingte NaCl-Ausscheidung über der Kontrollausscheidung ohne das Schleifendiuretikum ("Baseline") liegt. Beispielsweise ist die Menge eines Schleifendiuretikums, die der Wirkung einer Dosis von 10 mg Torasemid entspricht, eine Menge, die während der Wirkdauer dieses Schleifendiuretikums die gleiche Menge NaCl zur Ausscheidung bringt wie 10 mg Torasemid (siehe auch 31).

Dementsprechend werden Schleifendiuretika wie Piretanid gewöhnlich wie Torasemid in einer Menge von etwa 2,5 bis 15 mg, besonders bevorzugt von etwa 2,5 bis 14 mg und besonders bevorzugt von etwa 2,5 bis 12,5 mg pro Dosierungsseinheit eingesetzt. Furosemid liegt gewöhnlich in einer Menge von etwa 7,5 bis 45 mg, bevorzugt von etwa 7,5 bis 42 mg und ganz besonders bevorzugt von 7,5 bis 37,5 mg vor, und Bumetanid liegt gewöhnlich in einer Menge von 0,05 bis 0,3 mg, bevorzugt von 0,05 bis 0,25 mg und ganz besonders bevorzugt von 0,05 bis 0,2 mg vor.

Besonders bevorzugt werden Thiaziddiuretikum und Schleifendiuretikum in einer Menge und einem Mengenverhältnis verwendet, die über die Wirkdauer der Kombination, d.h. über die Zeit, in der die wirkstoffbedingte NaCl-Ausscheidung über der Kontrollausscheidung ohne die Wirkstoffkombination ("Baseline") liegt, ein K⁺/Na⁺-Ausscheidungsverhältnis von ≤ 0,3 bewirkt. Ein solches K⁺/Na⁺-Ausscheidungsverhältnis wird beispielsweise durch eine Kombination aus etwa 20 bis 30 mg Hydrochlorothiazid und etwa 7,5 bis 12,5 mg Torasemid erreicht, beispielsweise durch eine Wirkstoffkombination aus 25 mg Hydrochlorothiazid und 10 mg Torasemid oder entsprechende Mengen anderer Thiazid- und Schleifendiuretika.

Das erfindungsgemäße Kombinationspräparat kann die Wirkstoffe, wie oben erwähnt, zusammen oder getrennt voneinander enthalten, d.h. in Form einer einzigen oder getrennter Dosierungseinheiten. Bevorzugt liegt das erfindungsgemäße Kombinationspräparat in Form einer einzigen Dosierungseinheit vor, insbesondere als feste pharmazeutische Zusammensetzung, die die beiden Wirkstoffe enthält. Sie eignet sich zur oralen Verabreichung, beispielsweise in Form von Pulver, Granulaten, Tabletten, Dragees und Kapseln.

Das erfindungsgemäße Kombinationspräparat kann die Wirkstoffe, zusammen oder getrennt voneinander, gegebenenfalls in Mischung mit wenigstens einem pharmazeutisch verträglichen Hilfs- oder Zusatzstoff enthalten. Geeignete Hilfs- oder Zusatzstoffe sind beispielsweise Füllmittel wie Sucrose, Lactose, Cellulose, wie mikrokristalline Cellulose, Mannitol, Maltitol, Dextran, Stärken, wie Maisstärke, Agar, Alginate, Chitine, Chitosane, Pektine, Tragacanthgummi, Gummi arabicum, Gelatine, Casein, Albumin, synthetische oder halbsynthetische Polymere oder Glyceride, Gleitmittel wie Magnesiumstearat, Konservierungsmittel wie Paraben oder Sorbinsäure, Antioxidantien wie Ascorbinsäure, α-Tocopherol oder Cystein, Fließregulierungs- und Trocknungsmittel, beispielsweise hochdisperses Siliciumdioxid, Sprengmittel, beispielsweise Carboxymethylstärke-Natrium. Bindemittel, Verdickungsmittel, Süßmittel oder Geschmacksstoffe.

Die Herstellung der erfindungsgemäßen Kombinationspräparate kann auf dem Fachmann bekannte Art und Weise erfolgen. Beispielsweise können Zusammensetzungen, die die Wirkstoffe zusammen oder getrennt enthalten, hergestellt werden, indem man die Wirkstoffe sowie gegebenenfalls die pharmazeutisch verträglichen Hilfs- und Zusatzstoffe in Pulverform innig miteinander vermischt und vereint.

Die erfindungsgemäße fixe Kombination aus Thiaziddiuretikum und Schleifendiuretikum eignet sich besonders gut zur Behandlung cardiovaskulärer Erkrankungen wie Hypertonie und Herzinsuffizienz. Die vorliegende Erfindung betrifft daher auch die Verwendung eines Thiaziddiuretikums und eines Schleifendiuretikums als Wirkstoffe zur Herstellung eines Kombinationspräparats zur Behandlung von Hypertonie oder Herzinsuffizienz.

Die erfindungsgemäße Kombination aus Thiaziddiuretikum und Schleifendiuretikum eignet sich besonders zur Behandlung von primärer (essentieller) Hypertonie.

Auch sekundäre Hypertonien, beispielsweise die renale Hypertonie, können insbesondere bei einer nur leichten bis mäßigen Nierenfunktionsstörung mit einer GFR ≥ 30 ml/min (Plasmacreatinin ≤ 2 mg/dl), wie sie bei dieser Patientengruppe immer wieder auftritt, gut mit der erfindungsgemäßen Kombination behandelt werden.

Die erfindungsgemäße Kombination aus Thiaziddiuretikum und Schleifendiuretikum eignet sich außerdem sehr gut zur Behandlung von chronischen und subchronischen Formen von Herzinsuffizienz mit einem unverminderten effektiven arteriellen Blutvolumen (EABV) bzw. einem Herzindex (Herzminutenvolumen pro Körperoberfläche [1 min⁻¹ m⁻²]) von ≥ 2 1 min⁻¹ m⁻² (nach 32 und 33), sowie zur Stabilisierung des Herzens nach Rekompensation des akuten Herzversagens.

Das erfindungsgemäße Kombinationspräparat wird dem zu behandelnden Subjekt, insbesondere Menschen, bevorzugt einbis zweimal, vorzugsweise einmal täglich verabreicht. Die genaue Dosierung für einen Patienten hängt beispielsweise von Alter, Geschlecht, Körpergewicht und Ernährung und vom Allgemeinzustand und vom zu behandelnden Zustand des Patienten ab. Die genaue Dosierung kann von einem erfahrenen Mediziner unter Berücksichtigung dieser und anderer Faktoren ohne Schwierigkeiten bestimmt werden.

Die erfindungsgemäße Kombination aus Thiazid- und Schleifendiuretikum kann gegebenenfalls auch in Kombination mit anderen pharmazeutischen Wirkstoffen verabreicht werden, die zur Behandlung von Hypertonie oder Herzinsuffizienz eingesetzt werden. Beispiele für solche Wirkstoffe sind ACE-Hemmer wie Captopril, Benazepril und Enalapril und AT1-Rezeptor-Antagonisten wie Candesartan, Irbesatan und Valsatan.

Die erfindungsgemäßen Kombinationspräparate haben den Vorteil, dass sie keine hohen Wirkungsspitzen ("Peak-Diurese") aufweisen und dass es nicht wie bei der Einzelgabe eines Schleifendiuretikums zu einem ausgeprägten Rebound kommt, d. h. einer postdiuretischen Phase, in der die NaCl-Ausscheidung unter der Kontrollausscheidung liegt. Die Wirkdauer des Schleifendiuretikums wird also durch das Thiazid zu einem günstigen Tagesprofil verlängert, wodurch die insbesondere bei Hochdruckpatienten erwünschte Einmalgabe möglich wird. Die Kombination aus Thiaziddiuretikum und Schleifendiuretikum hat ferner den Vorteil, dass sie hinsichtlich der Kalium-Magnesium-Exkretion, normiert auf die NaCl-Ausscheidung, überraschenderweise signifikant besser ist als die herkömmliche Kombination aus Thiazid und Antikaliuretikum. Dabei bleibt die erwünschte hypocalciurische Wirkung des Thiazids trotz Anwesenheit des Schleifendiuretikums erhalten. Auf diese Weise kann einer unerwünschten Hypokalämie, Hypomagnesämie und/oder Hypocalcämie und den damit verbundenen Nebenwirkungen wie Muskelschwäche, Obstipation, Knochenabbau und Herzrhythmusstörungen wirksam vorgebeugt werden. Darüber hinaus kann bei dieser Kombination ein ACE-Hemmer oder ein AT₁-Antagonist komediziert werden.

Mit den erfindungsgemäßen Wirkstoffkombinationen werden daher Medikamente bereitgestellt, die bei effektiver Natriurese nur zu geringen Kalium- und Magnesiumverlusten führen und daher bei guter Wirksamkeit ein verbessertes Nutzen-NebenwirkungsProfil besitzen. Die erfindungsgemäße Wirkstoffkombination eignet sich daher hervorragend zur Langzeit- und Dauerbehandlung.

Die vorliegende Erfindung wird nachfolgend anhand von Beispielen und Figuren näher beschrieben.

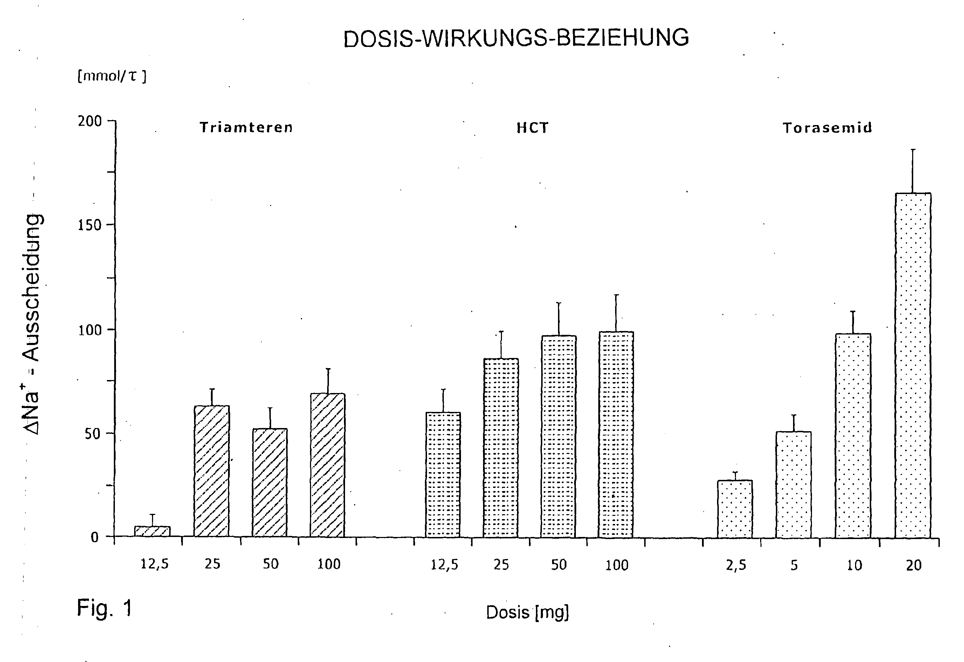

- Fig. 1: zeigt die Nettoausscheidung (= Gesamtausscheidung abzüglich der in der gleichen Zeit am Vortag ermittelten Kontrollausscheidung) von Na⁺ (ΔNa⁺) während der Wirkdauer (τ)(= Zeit, in der die arzneistoffinduzierte Ausscheidung über der Kontrollausscheidung liegt) als Funktion der verabreichten Dosis Triamteren, Hydrochlorothiazid (HCT) und Torasemid;

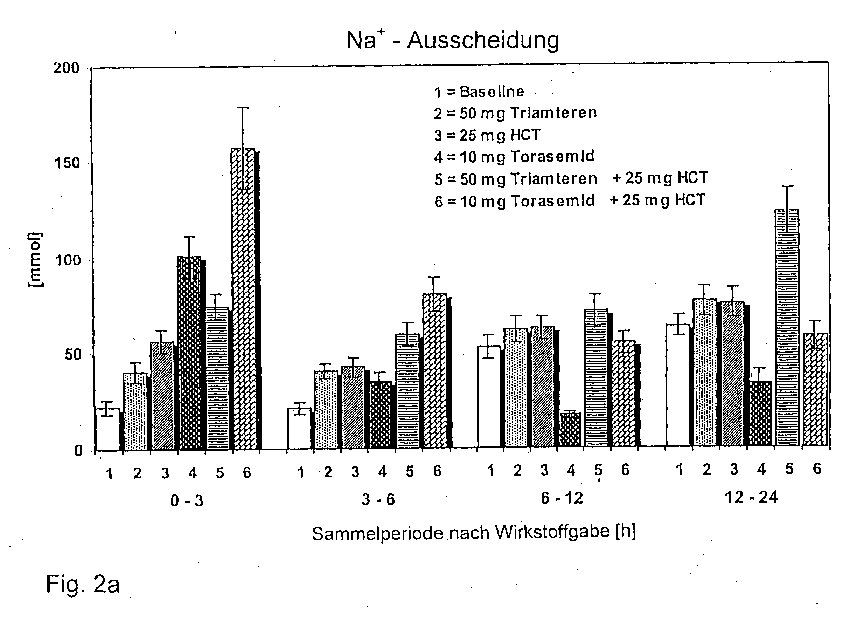

- Fig. 2a-d: zeigen das zeitliche Ausscheidungsprofil von Na⁺ (2a)und K⁺ (2b) , kumuliert für unterschiedliche Perioden nach Wirkstoffgabe, sowie das Ausscheidungsverhältnis von K⁺/Na⁺ (2c) und Ca²⁺/Na⁺ (2d) für die jeweilige Sammelperiode im Urin;

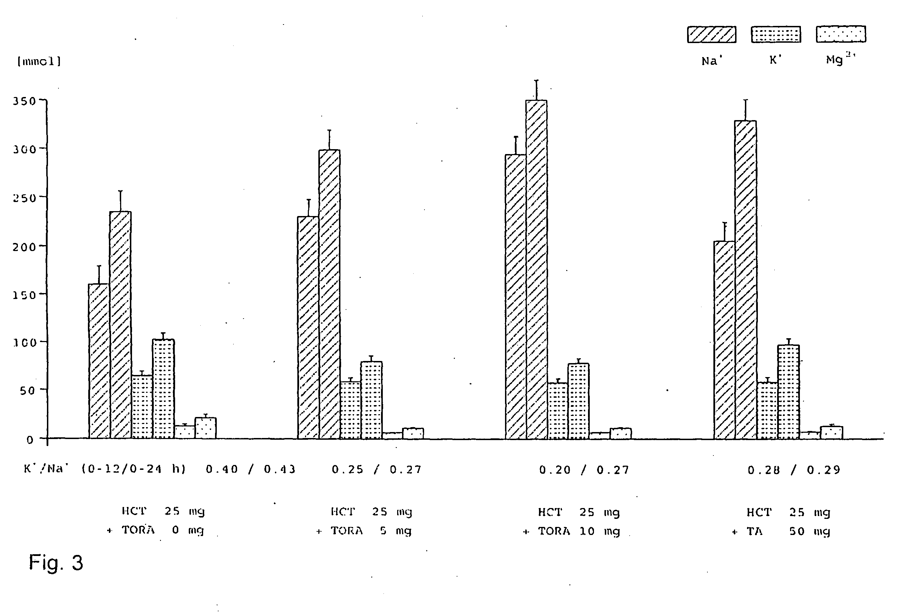

- Fig. 3: zeigt die kumulative Gesamtausscheidung von Na⁺, K⁺ und Mg², für die Periode von Stunde 0 -12 (linke Säule des Säulenpaars) und von Stunde 0 -24 (rechte Säule) nach Gabe von Hydrochlorothiazid (HCT) und Torasemid (TORA) im Vergleich zur HCT-Monotherapie. Als Vergleich sind auch die Daten angegeben, die mit der konventionellen Kombination von HCT und Triamteren (TA) erhalten wurden. Unter den Säulen ist das Ausscheidungsverhältnis Na⁺/K⁺ angegeben, bestimmt für Stunde 0 - 12 (0 - 24) nach Gabe des jeweiligen Regimes.

### Beispiele

### 1. Methoden

Die ethisch bewilligte Studie erfolgte an 12 gesunden Freiwilligen (Alter 28 - 48 Jahre, 6 männlich, 6 weiblich), die schriftlich ihre Zustimmung zu der Studie erteilt hatten. Vor der Gabe eines Wirkstoffs wurde eine klinische Untersuchung der Teilnehmer durchgeführt. Die Teilnehmer erhielten eine Standarddiät, wobei sie drei Tage vor Beginn der Studie und während der gesamten Studie eine definierte Menge Flüssigkeit (1,5 1/Tag) und Salz (6 g NaCl; 100 mmol Na⁺/Tag) aufnahmen.

Entsprechend früheren Studien (35) wurde vor der Behandlungsperiode, d.h. von Stunde -24 bis -12 und von Stunde -12 bis 0 vor oraler Wirkstoffgabe (8 Uhr morgens), Urin gesammelt. Ab dann wurde bis Stunde 6 in Abständen von 2 Stunden und dann von Stunde 6 bis 9, 9 bis 12 und 12 bis 24 nach Wirkstoffgabe Urin gesammelt. Ein Aliquot des definierten Volumens der jeweiligen Sammelperiode wurde bis zur Analyse auf Na⁺, K⁺, Cl⁻, Ca², Mg²⁺ und Kreatinin mit einem "MODULAR" Analyzer (Roche Comp., Schweiz) eingefroren.

Für alle Daten sind die Mittelwerte (± S.E.M.) angegeben. Die Diuretika-induzierte "Netto-Ausscheidung" ist durch die Differenz zwischen Gesamtausscheidung und Kontrollausscheidung ("Baseline"), die für die jeweilige Periode vor der Behandlung erhalten wurde, definiert. Die Wirkung der diuretischen Monotherapie wird mittels ANOVA mit der Kömbinationstherapie verglichen.

### 2. Ergebnisse

### A. Dosis-Wirkungs-Studien

Dosis-Wirkungs-Studien von Hydrochlorothiazid (HCT) und Triamteren (TA) bestätigen allgemein bekannte Beobachtungen, dass diese Klassen von Diuretika ihre maximale natriuretische Wirkung bereits bei üblichen Dosen von 25 mg HCT bzw. 50 mg TA erreichen (Fig. 1). Die durch 25 mg HCT induzierte kumulative Na⁺-Ausscheidung über 12 Stunden (der Zeit bis zum erneuten Erreichen der Kontrollausscheidung) unterschied sich jedoch nicht signifikant von derjenigen, die mit 50 mg erhalten wurde. Der enge Bereich nahezu linearer Dosis-Wirkungs-Beziehung charakterisiert diese Diuretika als "low ceiling" Diuretika.

Auf der anderen Seite hat das "high ceiling"-Schleifendiuretikum Torasemid (TORA) im Verhältnis zu seiner kurzen Wirkungsdauer (6 Stunden) einen breiteren Bereich linearer Dosis-Wirkungs-Beziehung. Es ist jedoch bekannt, dass diese Klasse von Diuretika deutlich weniger wirksam ist als Thiazide, wenn die natriuretische Wirkung einer täglichen Dosis auf 24 Stunden bezogen wird (33, 34).

### B. Diuretische Wirkungen auf die Ausscheidung von Na⁺, K⁺, Mg²⁺ und Ca²⁺.

### 1. Monotherapie

Die natriuretische Wirkung von üblichen Dosen der Diuretika wurde anschließend mit der Ausscheidung von K⁺, Mg²⁺, und Ca²⁺ in Beziehung gesetzt, definiert für die unterschiedlichen Perioden nach Wirkstoffgabe. Figur 2a zeigt, dass 10 mg TORA während der ersten 3 Stunden bei weitem die größte Zunahme in der Na-Ausscheidung zur Folge hatten, gefolgt von 25 mg HCT und dann 50 mg TA. Während der Periode von Stunde 3 - 6 ging dieser Unterschied verloren und während der Perioden von Stunde 6 - 12 und Stunde 12 - 24 zeigten die Schleifendiuretika den allgemein bekannten "Rebound", d.h. die Na⁺-Ausscheidung fiel unter den Wert vor Arzneistoffgabe. HCT induzierte nur über die ersten 3 Stunden eine größere Na⁺-Auscheidung als TA.

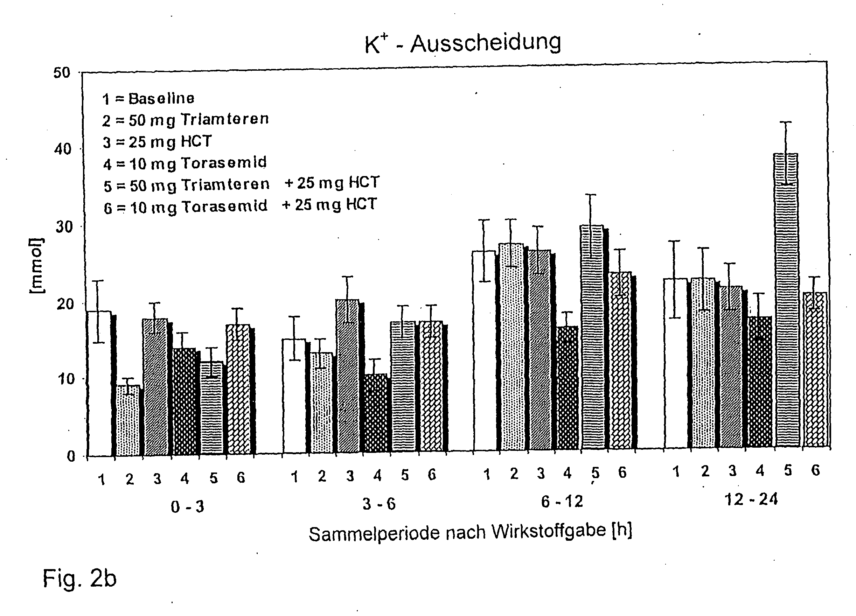

Die von HCT induzierte Gesamtausscheidung an K⁺ war größer als diejenige, die der Gabe des Schleifendiuretikums folgte. Dies galt insbesondere für die Wirkdauer des Thiazids (Stunde 0 - 12) sowie während dessen postdiuretischer Periode (Stunde 12 -24). Nur während der Periode von Stunde 0 - 3 induzierte TA weniger K⁺-Verlust als HCT oder TORA (Fig. 2b).

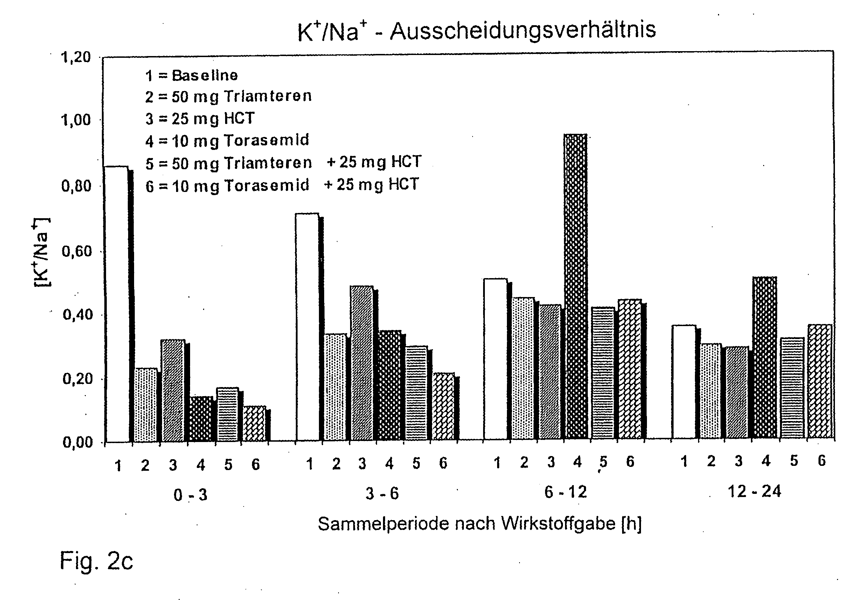

Wenn die K⁺-Ausscheidung - wie früher definiert (35, 36) - mit der Na⁺-Auscheidung ins Verhältnis gesetzt wurde, wurde deutlich, dass das K⁺/Na⁺-Ausscheidungsverhältnis von HCT während der ersten drei Stunden nach Wirkstoffgabe nahezu das dreifache des Schleifendiuretikums betrug (Fig. 2c). Das K⁺/Na⁺-Ausscheidungsverhältnis von TORA war erst nach der Wirkdauer des Schleifendiuretikums (Stunde 6 - 24) am größten, da die Na⁺-Ausscheidung dann unter dem Grundwert lag. Überraschenderweise war das kaliumsparende Diuretikum TA nicht besser als das Schleifendiuretikum, was die K⁺-Ausscheidung insgesamt (Stunde 0 - 24) (Fig. 2b) sowie die auf die Na⁺-Ausscheidung bezogene K⁺-Ausscheidung (Stunde 0 - 6) (Fig. 2c) betrifft.

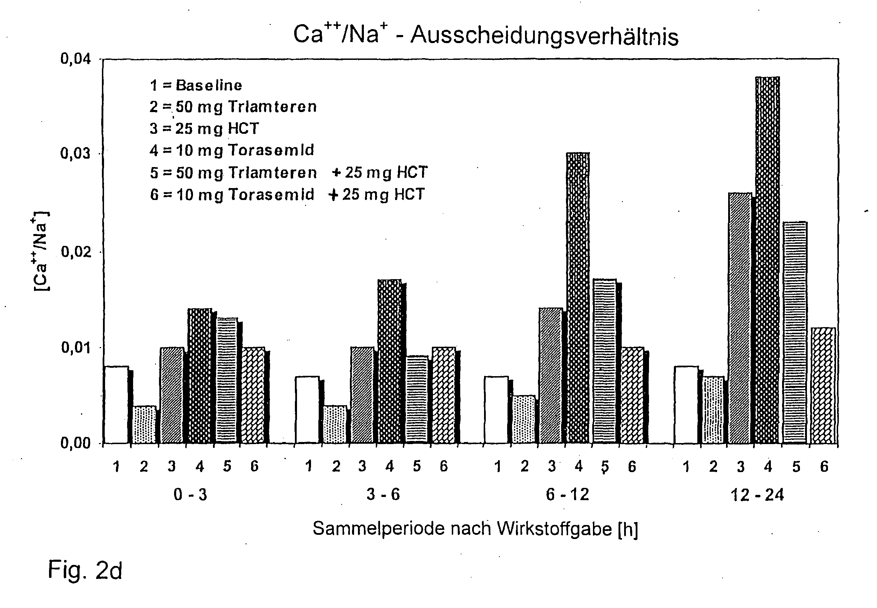

Die Ausscheidung von Mg²⁺ im Verhältnis zu der von K⁺ änderte sich nach Gabe von Thiazid und Schleifendiuretikum nicht signifikant (Fig. 3). Mit anderen Worten ist das Schleifendiuretikum weniger magnesiuretisch als das Thiazid. Auf der anderen Seite war.die Ca²⁺-Ausscheidung im Verhältnis zur Na⁺-Ausscheidung in Übereinstimmung mit früheren Beobachtungen (35, 36) bei dem Schleifendiuretikum größer als mit dem Thiazid (Fig. 2d).

### 2. Diuretikakombinationen

Die kombinierte Verabreichung von HCT mit dem Schleifendiuretikum oder dem kaliumsparenden Diuretikum steigerte die natriuretische Wirkung für die Periode von Stunde 0 - 6 signifikant (Fig. 2a). Die HCT/TORA-Kombination lieferte eine supra-additive natriuretische Wirkung, d.h. die Netto-Na⁺-Ausscheidung (Gesamtausscheidung minus Grundwert) war bei gemeinsamer Verabreichung der Diuretikakombination signifikant höher als die Summe jeder für sich allein verabreichten Diuretikaklasse. In Kombination mit dem kaliumsparenden Diuretikum induzierte HCT - wenn überhaupt - nur eine additive natriuretische Wirkung.

Am überraschendsten war die Ausscheidung von K⁺, Mg²⁺ und Ca²⁺ im Verhältnis zur natriuretischen Wirksamkeit der Diuretikakombination. Wie in Fig. 3 gezeigt, war die Gesamtausscheidung an K⁺ und Mg²⁺ sowohl für die Wirkdauer (Stunde 0 - 12) als auch über die Stunden 0 - 24 bei gemeinsamer Verabreichung mit TORA geringer als mit HCT allein. Diese Änderung läßt sich am besten quantifizieren, indem man die K⁺-Ausscheidung mit der Na⁺-Ausscheidung in Beziehung setzt, die durch 5 mg TORA von 0,40 (0,43) auf 0,25 (0,27) (p<0,01) verringert wurde. Diese "K⁺-sparende Wirkung" von TORA war bei gemeinsamer Verabreichung mit 10 mg TORA mit HCT noch ausgeprägter (Fig. 3).

Die Zugabe von TORA verringerte auch die HCT-induzierte Ausscheidung von Mg²⁺ (p<0,01). Außerdem lässt sich aus dem zeitlichen Verlauf der in Fig. 2 dargestellten Elekrolytausscheidung sehen, dass die hypocalciurische Wirkung von HCT erhalten blieb, wenn es mit dem Schleifendiuretikum kombiniert wurde (Fig. 2d).

Durch Vergleich der HCT/TORA-Kombination mit der herkömmlichen HCT/TA-Kombination (Fig. 3) wird deutlich, dass das Schleifendiuretikum dem kaliumsparenden Diuretikum gegenüber einer alleinigen Gabe von Thiaziden überlegen ist, und zwar sowohl was die gewünschte natriuretische Wirkung als auch was den unerwünschten K⁺- und Mg²⁺-verlust betrifft.

### Zitierte Literatur

1. Black WD, Shiner PT, Roman J. (1978) Severe electrolyte disturbances associated with metolazone and furosemide. South Med J 71: 381-385
2. Leiter L (1970) Combinations of diuretics in the treatment of edema. Am Heart J 80: 422
3. Gunstone RF, Wing AJ, Shani HGP, Njemo D, Sabuka EMW (1971) Clinical experience with metolazone in fifty-two African patients: synergy with furosemide. Postgrad med J 47: 789-793
4. Oleson KH (1971) The natriuretic effect of addition of quinethazone and furosemide in congestive heart failure. Acta Med Scand 190:229
5. Olesen KH, Sigurd B (1971) The supra-additive natriuretic effect by addition of quinethazone or bendroflumethiazide during long-term treatment with furosemide and spironolactone: permutation trial tests in patients with congestive heart failure. Acta Med Scand 199: 233-240
6. Asscher AW (1974) Treatment of frusemide resistant oedema with metolazone. Clin Trials J 4: 134-138
7. Sigurd B, Olesen KH, Wennevold A (1975) The supra-additive natruretic effect of addition of bendroflumethiazide and bumetanide in congestive heart failure. Am Heart J 86; 163-170
8. Ram CVS, Reichgott MJ (1977) Treatment of loop diuretic resistant edema by the addition of metolazone. Curr Ther Res 22: 686-691
9. Epstein M, Lepp BA, Hoffman DS, Levinson R (1977) Potentiatioin of furosemide by metolazone in refractory edema. Current Therapeutic Research 21:656-667
10. Furrer, J Hess OM, Kuhlmann U, Satz N, Siegenthaler W (1980) Furosemid und Metolazon: eine hochwirksame Diuretikakombination Schweiz Med Wschr 110: 1825-1829
11. Garin EH, Richard GA (1981) Edema resistant to furosemide and metolazone. Int J Pediatr Nephrol 2: 181-184
12. Ghose RR, Gupta SK (1981) Synergistic action of metolazone with loop diuretics. Br Med J 282: 1825-1829
13. Wollam G Tarazi R., Bravo E. and Dustan H. (1982) Diuretic potency of combined hydrochlorothiazide and furosemide therapy in patients with azotemia. Am J Med 72, 929-938
14. Oster JR, Epstein M, Smoller S (1983) Combined therapy with thiazide-type and loop diuretic agents for resistant sodium retention. Ann Intern Med 99: 405-406
15. Arnold WC (1984) Efficacy of metolazone and furosemide in children with furosemid resistant edema. Pediatrics 74: 872-875
16. Marone C., Muggli F., Lahn W., and Frey F.J. (1985) Pharmacokinetic and pharmacodynamic interaction between furosemide and metolazone in man. Eur J Clin Invest. 15: 253-257
17. Garin EH (1987) A comparison of combinations of diuretics in nephrotic edema. AJDD 141: 769-771
18. Channer KS, Richardson M, Crook R, Jones JV (1990) Thiazides with loop diurectis for severe congestive heart failure. Lancet 335: 922-923
19. Oimomi M, Takase S, and Saeki S (1990) Combination diuretic therapy for severe refractory nephrotic syndrome. Lancet 336: 1004-1005
20. Knauf H, Mutschler E (1993) Low dose segmental blockade of the nephron rather than high dose diuretic monotherapy. In "Diuretics IV. Chemistry Pharmacology and Clinical Applications" (J. Puschett and A. Greenberg, Eds.), pp 449-456. Elsevier Amsterdam.
21. Channer KS, McLean K., Lawson-Matthew P, and Richardson M (1994). Combination diuretic treatment in severe heart failure: A randomised controlled trial. Br Heart J 71: 146-150
22. Fliser D, Schroter M, Neubeck M, and Ritz E. (1994) Coadministration of thiazides increases the efficacy of loop diuretics even in patients with advanced renal failure. Kidney Int. 46: 482-488
23. Knauf H, Mutschler E (1995) Diuretic effectiveness of hydrochlorothiazide and furosemide alone and in combination in chronic renal failure. J Cardiovasc Pharmacol 26: 394-400
24. Ellison DH (1991) The physiologic basis of diuretic synergism: Its role in treating diuretic resistance. Ann Int Med. 114: 886-894
25. Brater DC (1994) Diuretic Resistance: Mechanisms and Therapeutic Strategies. Cardiology 84 (suppl 2): 57-67
26. Kaissling B, Bachmann S, and Kriz W (1985) Structural adaptation of the distal convoluted tubule to prolonged furosemide treatment. Am J Physiol 248, F374-F381
27. H. Knauf et al. (1997) Therapieempfehlungen für Patienten mit "diuretikaresistenten" hydropischen Zuständen. Notabene Medici 7, 350-351
28. Anderson J, Godfrey BE, Hill DM, Munro-Faure AD, Sheldon J (1971) A comparison of the effects of hydrochlorothiazide and of furosemide in the treatment of hypertensive patients. Quarterly Journ of Med XL: 541-560
29. Bariso CR, Hanenson IB, Gaffney TE (1970) A comparison of the antihypertensive effects of furosemide and chlorothiazide. Curr Ther Res 12: 333-340
30. McMahon FG (1990) Management of essential hypertension. The once-a-day era. Futura, New York, S. 287-378
31. Knauf H und Mutschler E (1996) Das Wirkprofil von Diuretika. Nieren- und Hochdruckkrankheiten 25(4): 162-170
32. Roskamm H, Reindel H (1982) Herzkrankheiten. Springer Verlag Berlin, S. 361
33. Buchwalsky R (1985) Einschwemmkatheter. In: Beiträge zur Kardiologie Band 29, S. 143, perimed Fachbuchverlagsgesellschaft Erlangen
34. Leary WP und Reyes AJ (1993) Low-dose segmental blockade of the nephron rather than high-diuretic monotherapy. In: Diuretics IV. Chemistry Pharmacology, and Clinical Applications. Puschett J, Greenberg A (Hrsg.) Elsevier, Amsterdam, S. 449-456
35. Knauf H und Mutschler E (1990) Saluretic effects of the loop diuretic torasemid in chronic renal failure. Interdependence of electrolyte excretion. Eur J Clin Pharmacol 39: 337-343
36. Velazquez H, Knauf H und Mutschler E (1995) Thiazide diuretics. In: Diuretics. Greger RF, Knauf H, Mutschler E (Hrsg.), Springer, Berlin, New York, S. 275-334

## Patentansprüche

1. Kombinationspräparat, umfassend, zusammen oder getrennt voneinander, ein Thiaziddiuretikum und ein Schleifendiuretikum, wobei das Thiaziddiuretikum ausgewählt ist aus:
Hydrochlorothiazid in einer Menge von 5 bis 50 mg;
Hydroflumethiazid in einer Menge von 5 bis 50 mg;
Benzthiazid in einer Menge von 5 bis 50 mg;
Chlorthalidon in einer Menge von 5 bis 50 mg;
Quinethazon in einer Menge von 5 bis 50 mg;
Bendroflumethiazid in einer Menge von 0,5 bis 5 mg;
Methyclothiazid in einer Menge von 0,5 bis 5 mg;
Indapamid in einer Menge von 0,25 bis 1,25 mg;
Polythiazid in einer Menge von 0,2 bis 2 mg; und
Trichlormethiazid in einer Menge von 0,2 bis 2 mg;
und das Schleifendiuretikum ausgewählt ist aus:
Torasemid in einer Menge von 2,5 bis 15 mg;
Piretanid in einer Menge von 2,5 bis 15 mg;
Furosemid in einer Menge von 7,5 bis 37,5 mg; und
Bumetanid in einer Menge von 0,05 bis 0,3 mg.
sowie pharmazeutisch verträglichen Derivaten dieser Thiaziddiuretika und Schleifendiuretika.

2. Kombinationspräparat nach Anspruch 1, wobei das Thiaziddiuretikum ausgewählt ist aus:
Hydrochlorothiazid in einer Menge von 10 bis 30 mg;
Hydroflumethiazid in einer Menge von 10 bis 30 mg;
Benzthiazid in einer Menge von 10 bis 30 mg;
Chlorthalidon in einer Menge von 10 bis 30 mg;
Quinethazon in einer Menge von 10 bis 30 mg;
Bendroflumethiazid in einer Menge von 1 bis 3,0 mg;
Methyclothiazid in einer Menge von 1 bis 3,0 mg;
Indapamid in einer Menge von 0,5 bis 1,5 mg;
Polythiazid in einer Menge von 0,4 bis 1,2 mg; und
Trichlormethiazid in einer Menge 0,4 bis 1,2 mg;
und das Schleifendiuretikum ausgewählt ist aus:
Torasemid in einer Menge von 2,5 bis 12,5 mg;
Piretanid in einer Menge von 2,5 bis 12,5 mg;
Furosemid in einer Menge von 7,5 bis 37,5 mg; und
Bumetanid in einer Menge von 0,05 bis 0,2 mg.
sowie pharmazeutisch verträglichen Derivaten dieser Thiaziddiuretika und Schleifendiuretika.

3. Kombinationspräparat nach Anspruch 1 oder 2, wobei das Thiaziddiuretikum Hydrochlorothiazid ist und das Schleifendiuretikum Torasemid ist.

4. Kombinationspräparat nach einem der Ansprüche 1 bis 3 in Form einer festen pharmazeutischen Zusammensetzung, welche das Thiaziddiuretikum und das Schleifendiuretikum umfasst.

5. Kombinationspräparat nach einem der Ansprüche 1 bis 3, wobei das Thiaziddiuretikum und das Schleifendiuretikum getrennt voneinander vorliegen.

6. Kombinationspräparat nach einem der Ansprüche 1 bis 5, welches wenigstens einen pharmazeutisch verträglichen Hilfs- oder Zusatzstoff umfasst.

7. Kombinationspräparat nach Anspruch 6, wobei der pharmazeutisch verträgliche Hilfs- oder Zusatzstoff ausgewählt ist aus Füllstoffen, Gleitmitteln, Konservierungsmitteln, Antioxidantien, Sprengmitteln, Bindemitteln, Verdickungsmitteln, Süßmitteln oder Geschmacksstoffen.

8. Kombinationspräparat nach einem der Ansprüche 1 bis 7 in Form von Pulver, Granulaten, Tabletten, Dragees oder Kapseln.

9. Verwendung eines Thiaziddiuretikums und eines Schleifendiuretikums zur Herstellung eines Kombinationspräparats zur Behandlung von Hypertonie oder Herzinsuffizienz, wobei das Thiaziddiuretikum ausgewählt ist aus:
Hydrochlorothiazid in einer Menge von 5 bis 50 mg;
Hydroflumethiazid in einer Menge von 5 bis 50 mg;
Benzthiazid in einer Menge von 5 bis 50 mg;
Chlorthalidon in einer Menge von 5 bis 50 mg;
Quinethazon in einer Menge von 5 bis 50 mg;
Bendroflumethiazid in einer Menge von 0,5 bis 5 mg;
Metolazon in einer Menge von 0,5 bis 5 mg;
Methyclothiazid in einer Menge von 0,5 bis 5 mg;
Indapamid in einer Menge von 0,25 bis 1,25 mg;
Polythiazid in einer Menge von 0,2 bis 2 mg; und
Trichlormethiazid in einer Menge von 0,2 bis 2 mg;
und das Schleifendiuretikum ausgewählt ist aus:
Torasemid in einer Menge von 2,5 bis 15 mg;
Piretanid in einer Menge von 2,5 bis 15 mg;
Furosemid in einer Menge von 7,5 bis 45 mg; und
Bumetanid in einer Menge von 0,05 bis 0,3 mg.
sowie pharmazeutisch verträglichen Derivaten dieser Thiaziddiuretika und Schleifendiuretika.

10. Verwendung nach Anspruch 9 zur Herstellung eines Kombinationspräparats zur Behandlung von primärer Hypertonie.

11. Verwendung nach Anspruch 9 zur Herstellung eines Kombinationspräparats zur Behandlung von renaler Hypertonie mit Plasmacreatinin ≤ 2 mg/dl.

12. Verwendung nach Anspruch 9 zur Herstellung eines Kombinationspräparats zur Behandlung von Herzinsuffizienz mit unvermindertem effektivem arteriellen Blutvolumen (EABV).

13. Verwendung nach einem der Ansprüche 9 bis 12 zur Herstellung eines Kombinationspräparats zur Behandlung von Hypertonie oder Herzinsuffizienz in Kombination mit einem ACE-Hemmer oder AT1-Rezeptor-Antagonisten.

14. Verwendung nach einem der Ansprüche 9 bis 13 zur Herstellung eines Kombinationspräparats zur Langzeit- oder Dauertherapie.

## Claims

1. Combination preparation comprising, combined or separated from each other, a thiazide diuretic and a loop diuretic, wherein the thiazide diuretic is selected from:
hydrochlorothiazide in an amount of from 5 to 50 mg;
hydroflumethiazide in an amount of from 5 to 50 mg;
benzthiazide in an amount of from 5 to 50 mg;
chlorthalidone in an amount of from 5 to 50 mg;
quinethazone in an amount of from 5 to 50 mg;
bendroflumethiazide in an amount of from 0.5 to 5 mg;
methychlothiazide in an amount of from 0.5 to 5 mg;
indapamide in an amount of from 0.25 to 1.25 mg;
polythiazide in an amount of from 0.2 to 2 mg; and
trichlormethiazide in an amount of from 0.2 to 2 mg;
and the loop diuretic is selected from:
torasemide in an amount of from 2.5 to 15 mg;
piretanide in an amount of from 2.5 to 15 mg;
furosemide in an amount of from 7.5 to 37.5 mg; and
bumetanide in an amount of from 0.05 to 0.3 mg.
and from pharmaceutically acceptable derivatives of said thiazide diuretics and loop diuretics.

2. Combination preparation according to Claim 1, wherein the thiazide diuretic is selected from:
hydrochlorothiazide in an amount of from 10 to 30 mg;
hydroflumethiazide in an amount of from 10 to 30 mg;
benzthiazide in an amount of from 10 to 30 mg;
chlorthalidone in an amount of from 10 to 30 mg;
quinethazone in an amount of from 10 to 30 mg;
bendroflumethiazide in an amount of from 1 to 3.0 mg;
methychlothiazide in an amount of from 1 to 3.0 mg;
indapamide in an amount of from 0.5 to 1.5 mg;
polythiazide in an amount of from 0.4 to 1.2 mg;
trichlormethiazide in an amount of from 0.4 to 1.2 mg;
and the loop diuretic is selected from:
torasemide in an amount of from 2.5 to 12.5 mg;
piretanide in an amount of from 2.5 to 12.5 mg;
furosemide in an amount of from 7.5 to 37.5 mg; and
bumetanide in an amount of from 0.05 to 0.3 mg.
and from pharmaceutically acceptable derivatives of said thiazide diuretics and loop diuretics.

3. Combination preparation according to Claim 1 or 2, wherein the thiazide diuretic is hydrochlorothiazide and the loop diuretic is torasemide.

4. Combination preparation according to any one of Claims 1 to 3 in the form of a solid pharmaceutical composition comprising the thiazide diuretic and the loop diuretic.

5. Combination preparation according to any one of Claims 1 to 3, wherein the thiazide diuretic and the loop diuretic are separated from each other.

6. Combination preparation according to any one of Claims 1 to 5 comprising at least one pharmaceutically acceptable excipient or additive.

7. Combination preparation according to Claim 6, wherein the pharmaceutically acceptable excipient or additive is selected from fillers, lubricants, preservatives, antioxidants, disintegrants, binders, thickening agents, sweeteners or flavouring agents.

8. Combination preparation according to any one of Claims 1 to 7 in the form of a powder, granules, tablets, coated tablets or capsules.

9. Use of a thiazide diuretic and a loop diuretic for the manufacture of a combination preparation for the treatment of hypertension or heart failure, wherein the thiazide diuretic is selected from:
hydrochlorothiazide in an amount of from 5 to 50 mg;
hydroflumethiazide in an amount of from 5 to 50 mg;
benzthiazide in an amount of from 5 to 50 mg;
chlorthalidone in an amount of from 5 to 50 mg;
quinethazone in an amount of from 5 to 50 mg;
bendroflumethiazide in an amount of from 0.5 to 5 mg;
metolazone in an amount of from 0.5 to 5 mg;
methychlothiazide in an amount of from 0.5 to 5 mg;
indapamide in an amount of from 0.25 to 1.25 mg;
polythiazide in an amount of from 0.2 to 2 mg; and
trichlormethiazide in an amount of from 0.2 to 2 mg;
and the loop diuretic is selected from:
torasemide in an amount of from 2.5 to 15 mg;
piretanide in an amount of from 2.5 to 15 mg;
furosemide in an amount of from 7.5 to 45 mg; and
bumetanide in an amount of from 0.05 to 0.3 mg.
and from pharmaceutically acceptable derivatives of said thiazide diuretics and loop diuretics.

10. Use according to Claim 9 for the manufacture of a combination preparation for the treatment of primary hypertension.

11. Use according to Claim 9 for the manufacture of a combination preparation for the treatment of renal hypertension with plasma creatinine ≤ 2 mg/dl.

12. Use according to Claim 9 for the manufacture of a combination preparation for the treatment of heart failure with unreduced effective arterial blood volume (EABV).

13. Use according to any one of Claims 9 to 12 for the manufacture of a combination preparation for the treatment of hypertension or heart failure in combination with an ACE inhibitor or an AT1 receptor antagonist.

14. Use according to any one of Claims 9 to 13 for the manufacture of a combination preparation for long-term therapy or permanent therapy.

## Revendications

1. Préparation combinée, comprenant, conjointement ou séparés l'un de l'autre, un diurétique thiazidique et un diurétique de l'anse, le diurétique thiazidique étant choisi parmi :
l'hydrochlorothiazide en une quantité de 5 à 50 mg ;
l'hydrofluméthiazide en une quantité de 5 à 50 mg ;
le benzothiazide en une quantité de 5 à 50 mg ;
la chlorothalidone en une quantité de 5 à 50 mg ;
la quinéthazone en une quantité de 5 à 50 mg ;
le bendrofluméthiazide en une quantité de 0,5 à 5 mg ;
le méthylclothiazide en une quantité de 0,5 à 5 mg ;
l'indapamide en une quantité de 0,25 à 1,25 mg ;
le polythiazide en une quantité de 0,2 à 2 mg ; et
le trichlorométhiazide en une quantité de 0,2 à 2 mg ;
et le diurétique de l'anse étant choisi parmi :
le torasémide en une quantité de 2,5 à 15 mg ;
le pirétanide en une quantité de 2,5 à 15 mg ;
le furosémide en une quantité de 7,5 à 37,5 mg ; et
le bumétanide en une quantité de 0,05 à 0,3 mg.
et les dérivés pharmaceutiquement acceptables de ces diurétiques thiazidiques et diurétiques de l'anse.

2. Préparation combinée selon la revendication 1, dans laquelle le diurétique thiazidique est choisi parmi :
l'hydrochlorothiazide en une quantité de 10 à 30 mg ;
l'hydrofluméthiazide en une quantité de 10 à 30 mg ;
le benzothiazide en une quantité de 10 à 30 mg ;
la chlorothalidone en une quantité de 10 à 30 mg ;
la quinéthazone en une quantité de 10 à 30 mg ;
le bendrofluméthiazide en une quantité de 1 à 3,0 mg ;
le méthylclothiazide en une quantité de 1 à 3,0 mg ;
l'indapamide en une quantité de 0,5 à 1,5 mg ;
le polythiazide en une quantité de 0,4 à 1,2 mg ; et le trichlorométhiazide en une quantité de 0,4 à 1,2 mg ;
et le diurétique de l'anse est choisi parmi :
le torasémide en une quantité de 2,5 à 12,5 mg ;
le pirétanide en une quantité de 2,5 à 12,5 mg ;
le furosémide en une quantité de 7,5 à 37,5 mg ; et
le bumétanide en une quantité de 0,05 à 0,2 mg.
et les dérivés pharmaceutiquement acceptables de ces diurétiques thiazidiques et diurétiques de l'anse.

3. Préparation combinée selon la revendication 1 ou 2, dans laquelle le diurétique thiazidique est l'hydrochlorothiazide et le diurétique de l'anse est le torasémide.

4. Préparation combinée selon l'une des revendications 1 à 3, sous la forme d'une composition pharmaceutique solide qui comprend le diurétique thiazidique et le diurétique de l'anse.

5. Préparation combinée selon l'une des revendications 1 à 3, dans laquelle le diurétique thiazidique et le diurétique de l'anse se présentent sous forme séparée l'un de l'autre.

6. Préparation combinée selon l'une des revendications 1 à 5, qui comprend au moins un adjuvant ou un additif pharmaceutiquement acceptable.

7. Préparation combinée selon la revendication 6, dans laquelle l'adjuvant ou l'additif pharmaceutiquement acceptable est choisi parmi les charges, agents lubrifiants, agents conservateurs, agents anti-oxydants, agents de délitement, agents liants, agents épaississants, édulcorants ou agents de sapidité.

8. Préparation combinée selon l'une des revendications 1 à 7, sous la forme de poudres, de granulés, de comprimés, de dragées ou de gélules.

9. Utilisation d'un diurétique thiazidique et d'un diurétique de l'anse pour la fabrication d'une préparation combinée destinée au traitement de l'hypertension ou de l'insuffisance cardiaque, le diurétique thiazidique étant choisi parmi : l'hydrochlorothiazide en une quantité de 5 à 50 mg ;
l'hydrofluméthiazide en une quantité de 5 à 50 mg ;
le benzothiazide en une quantité de 5 à 50 mg ;
la chlorothalidone en une quantité de 5 à 50 mg ;
la quinéthazone en une quantité de 5 à 50 mg ;
le bendrofluméthiazide en une quantité de 0,5 à 5 mg ;
la métolazone en une quantité de 0,5 à 5 mg ;
le méthylclothiazide en une quantité de 0,5 à 5 mg ;
l'indapamide en une quantité de 0,25 à 1,25 mg ;
le polythiazide en une quantité de 0,2 à 2 mg ; et
le trichlorométhiazide en une quantité de 0,2 à 2 mg ;
et le diurétique de l'anse étant choisi parmi :
le torasémide en une quantité de 2,5 à 15 mg ;
le pirétanide en une quantité de 2,5 à 15 mg ;
le furosémide en une quantité de 7,5 à 45 mg ; et
le bumétanide en une quantité de 0,05 à 0,3 mg.
et les dérivés pharmaceutiquement acceptables de ces diurétiques thiazidiques et diurétiques de l'anse.

10. Utilisation selon la revendication 9, pour la fabrication d'une préparation combinée destinée à traiter l'hypertension primaire.

11. Utilisation selon la revendication 9, pour la fabrication d'une préparation combinée destinée au traitement de l'hypertension rénale associée à une créatinine plasmatique ≤ 2 mg/dl.

12. Utilisation selon la revendication 9, pour la fabrication d'une préparation combinée destinée au traitement de l'insuffisance cardiaque associée à une non diminution du volume sanguin artériel effectif (EABV).

13. Utilisation selon l'une des revendications 9 à 12, pour la fabrication d'une préparation combinée destinée au traitement de l'hypertension ou de l'insuffisance cardiaque en combinaison avec un inhibiteur de l'ACE ou avec des antagonistes du récepteur AT1.

14. Utilisation selon l'une des revendications 9 à 13, pour la fabrication d'une préparation combinée destinée à un traitement de longue durée ou un traitement permanent.
